(19) Europäisches Patentamt
European Patent Office
Office européen des brevets

(11) Veröffentlichungsnummer: **0 003 474**
**B1**

(12) **EUROPÄISCHE PATENTSCHRIFT**

(45) Veröffentlichungstag der Patentschrift: **25.11.81**

(51) Int. Cl.$^3$: **G 01 N 33/48**, C 07 G 7/00

(21) Anmeldenummer: **78101680.3**

(22) Anmeldetag: **14.12.78**

(54) **Verfahren zur Prothrombin-Bestimmung, dazu verwendetes Reagenz und Verfahren zur Herstellung eines Faktor-Xa-Präparats.**

(30) Priorität: **24.12.77 DE 2757992**

(43) Veröffentlichungstag der Anmeldung:
**22.08.79 Patentblatt 79/17**

(45) Bekanntmachung des Hinweises auf die Patenterteilung:
**25.11.81 Patentblatt 81/47**

(84) Benannte Vertragsstaaten:
**BE CH DE FR GB IT LU NL SE**

(56) Entgegenhaltungen:
**DD - A - 128 691**
**DE - A - 2 627 925**
**DE - A - 2 654 189**
**DE - B - 2 356 493**
**US - A - 3 486 981**

**ANGEWANDTE CHEMIE, 83. Jahrgang 1971, Nr. 3, Weinheim**
**N. HEIMBURGER u.a. "Blutgerinnung und Fibrinolyse"**
**Seiten 89 bis 102**

(73) Patentinhaber: **BOEHRINGER MANNHEIM GMBH**
**Sandhofer Strasse 112-132 Postfach 31 01 20**
***D-6800 Mannheim 31-Waldhof (DE)***

(72) Erfinder: **Kirchhof, Bruno, Dr.**
**Tannenhofallee 23e**
**D-4400 Münster (DE)**

**EP 0 003 474 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Prothrombin-Bestimmung, dazu verwendetes Reagenz und Verfahren zur Herstellung eines Faktor-Xa-Präparats

Die Erfindung betrifft ein Verfahren und ein Reagenz zur Prothrombin-Bestimmung in biologischen Material, wie z. B. Blutplasma.

Die Bestimmung des Prothrombinspiegels ist ein wichtiger klinischer Parameter für die Verlaufskontrolle der Antikoagulantientherapie. Ebenso bedeutungsvoll ist der Nachweis eines Faktor-II-Mangels, welcher sowohl angeboren als auch erworben, d. h. die Folge einer primären Grunderkrankung sein kann.

Ausgehend von natürlichem Thrombin-Substrat Fibrinogen ist man im Laufe der Entwicklung von Prothrombin-Bestimmungsmethoden auf die Verwendung von synthetischen chromogenen Substraten, z. B. Peptid-p-nitroanilid-Derivaten, übergegangen. Gewebethromboplastin wurde als Aktivator verwendet. Der Nachteil dieser Methode besteht z. B. darin, daß die optimale Aktivierungszeit mit der Prothrombinkonzentration variiert, und im Mangel an Spezifität. Bei anderen Substraten mußte man eine niedrigere Plasmakonzentration in der Aktivierungsmischung haben, um Prothrombin in normalem Plasma zu bestimmen. Durch eine solche hochgradige Plasma-Verdünnung war es aber nicht möglich, eine vollständige Aktivierung in Plasmen mit niedriger Prothrombinaktivität zu erreichen. Als Aktivatoren wurden auch Staphylokoagulase und Schlangengifte eingesetzt. Sie aktivieren Prothrombin direkt. Es hat sich aber gezeigt, daß diese Gifte das PIVKA-Prothrombin (PIVKA ist eine Kurzbezeichnung für *P*rotein *i*nduced by *v*itamin *K* *a*bsence; eine Synonymbezeichnung ist Decarboxyprothrombin), das sich während der Behandlung mit oralen Antikoagulantien bildet, zumindest teilweise auch aktivieren. Staphylokoagulase setzt ebenfalls dieses sog. PIVKA-Prothrombin um.

Der Erfindung liegt daher die Aufgabe zugrunde, ein Verfahren und ein Reagenz zur Bestimmung von Prothrombin in biologischem Material, wie Blutplasma, zu schaffen, welche einen Test mit weniger Fehlerquellen, erhöhter Präzision, Empfindlichkeit und besserer Reproduzierbarkeit ermöglichen. Ein solcher Test sollte spezifisch sein, d. h. nur Prothrombin und nicht auch PIVKA-Prothrombin erfassen. Dabei sollte alles Prothrombin schnell und vollständig in Thrombin übergeführt werden, ohne daß der hierzu erforderliche Aktivator in irgendeiner Form an der Farbreaktion teilnimmt.

Gelöst wird diese Aufgabe erfindungsgemäß durch ein Verfahren zur Bestimmung von Prothrombin in biologischem Material, wie im Blutplasma, durch Umwandlung in Thrombin, enzymatische Spaltung eines Thrombinsubstrats mit dem Thrombin und Messung eines Spaltprodukts, dadurch gekennzeichnet, daß zu dem zu untersuchenden biologischen Material Faktor Xa, vorzugsweise menschlicher Faktor Xa, zugegeben wird. Als weitaus am besten geeignet hat sich der menschliche Faktor Xa erwiesen. Es ist aber auch z. B. die Verwendung von Rinder-Faktor-Xa möglich.

Das Prinzip der Bestimmungsmethode läßt sich wie folgt erläutern: Thrombin spaltet z. B. aus Oligopeptiden, bei denen an die Carboxylgruppe des Arginins p-Nitroanilin als chromogene Gruppe durch Amidbindung angehängt ist, p-Nitroanilin ab:

$$\text{N-Tos-Gly-Pro-Arg-pNA} \xrightarrow[H_2O]{\text{Thrombin}} \text{N-Tos-Gly-Pro-Arg-OH+pNA.}$$

Als Thrombinsubstrate haben sich synthetische Peptid-p-nitroanilid-Derivate bewährt, insbesondere N-Tos-Gly-Pro-Arg-pNA und N-Cbz-Gly-Pro-Arg-pNA (Chromozym TH), Boehringer Mannheim GmbH, sowie H-D-Phe-Pip-Arg-pNA (S-2238) und Bz-Phe-Val-Arg-pNA (S-2160), Kabi. Die gelbe Farbe des freien p-Nitroanilins kann bei etwa 390 bis 410 nm photometrisch gemessen werden, wobei die pro Zeiteinheit freigesetzte Farbstoffmenge der Enzymaktivität proportional ist.

Bevorzugt werden als Puffer Tris- und/oder Imidazol-Puffer von etwa pH 8—9, dem gegebenenfalls Salzsäure und/oder Natriumchlorid zugesetzt wird.

Als Co-Reagentien zur Aktivierung des Prothrombins werden Phospholipide und Calciumchlorid eingesetzt.

Die Substratzugabe erfolgt nach vollständiger Umwandlung des Prothrombins in Thrombin in der Testlösung nach Zugabe des Faktors Xa.

Der erfindungsgemäße Faktor Xa ist gut zugänglich durch ein einfaches Gewinnungsverfahren, welches ebenfalls Gegenstand der Erfindung ist. Dieses Verfahren ist dadurch gekennzeichnet, daß man Plasma, vorzugsweise menschliches Plasma, mit einem Prothombinaktivator behandelt, nach Zentrifugieren mit einem Proteinadsorbens versetzt, das Präzipitat mit Proteinelutionsmittel eluiert und das Eluat mit einem Faktor-X-Aktivator und gegebenenfalls einem löslichen Calcium-Salz versetzt. Es ist vorteilhaft als Prothrombinaktivator Echis-carinatus-venom einzusetzen, geeignet sind aber auch Schlangengifte, wie Taipan snake venom (Oxyuranus scutellatus) und Trypsin.

Das nach der Behandlung mit dem Prothrombinaktivator erhaltene Gerinnsel, welches abzentrifugiert wird, besteht teilweise aus Fibrinogen, Antithrombin und Thrombin.

Als Proteinadsorbens kommt vorzugsweise Bariumsulfat, -citrat und -oxalat in Frage, es eignen sich aber auch Aluminiumhydroxid, DEAE-Sephadex und QAE-Sephadex; als Proteinelutionsmittel können beispielsweise eine wäßrige Trinatriumcitrat-Lösung, Phosphatpuffer oder eine Natriumchlorid-

Lösung verwendet werden. Als Faktor-X-Aktivator ist vor allem Russel's-viper-venom geeignet. Als lösliches Calcium-Salz ist Calciumchlorid bevorzugt. Für den Fall, daß im Eluat nach Behandeln mit dem Proteinelutionsmittel Bestandteile vorhanden sind, die sich auf das Gerinnungssystem negativ auswirken, beispielsweise Calcium-bindende Komponenten, wie Citrat-Ionen, wenn das Proteinelutionsmittel Natriumcitrat ist, ist es erforderlich, einen Dialyseschritt einzuschalten. Im genannten Fall z. B. führt man eine Dialyse gegen eine gepufferte physiologische Kochsalzlösung (Michaelispuffer) bei niedriger Temperatur, d. h. etwa 4°C, durch. Nach der Behandlung mit dem Faktor-X-Aktivator und Zugabe der Calcium-Ionen-haltigen Lösung wird das Präparat vor der Verwendung bis zur vollständigen Umwandlung von Faktor X in Faktor Xa bei niedriger Temperatur, vorzugsweise 4°C, stehengelassen. Es können weitere Schritte zur Erzielung eines hochgereinigten Faktor Xa angefügt werden. Diese weiteren Reinigungsschritte können bestehen aus Gelfiltration mit einem Molekularsieb, das den Bereich von 50.000—100.000 differenziert, z. B. Sephadex® G 100, Biogel® P 100, wobei mit Natriumacetat-Puffer (0,4 m, pH 7) eluiert werden kann. Weiterhin sind möglich eine Ammoniumsulfatpräzipitation (z. B. 45- bis 55%ige Lösung, pH 6—8), eine Ionenaustauscherchromatographie mit DEAE-Sephadex® A 50, DEAE-Cellulose oder QAE-Sephadex®/Cellulose, wobei als Puffer Na/K-Phosphat (z. B. 0,02 m, pH 6,8) oder ein Gradient 0,1—1,0 m NaCl verwendet werden können. Auch eine Hydroxyapatit-Behandlung (Phosphatpuffer 0,2—0,5 m, pH 6,8) sowie eine präparative Elektrophorese und evtl. eine Ultrazentrifugation sind möglich. Vorteilhaft werden die angegebenen Methoden kombiniert. Der so hochgereinigte Faktor Xa kann in den Test eingesetzt werden, in Form einer Lösung, z. B. in Veronal- bzw. Michaelispuffer, in physiologischer Kochsalzlösung und im Testpuffer. Zur Verwendung im erfindungsgemäßen Test kann auf diesen Reinigungsschritt jedoch verzichtet werden und das wie oben beschrieben angereicherte Faktor-Xa-Präparat eingesetzt werden. In der DE—OS 26 54 189 ist zwar ein Verfahren zur Herstellung eines Faktor-Xa-Präparats beschrieben. Hieraus läßt sich das erfindungsgemäße Verfahren nicht ohne erfinderische Tätigkeit herleiten. Das dort beschriebene Verfahren ergibt keinerlei Hinweis auf eine Beseitigung des Prothrombins (Faktor II) an einer bestimmten Stelle des Herstellungsverfahrens; dieses Verfahren führt zu einem Präparat, welches neben Faktor X noch Faktor II enthält. Die überraschende Verbesserung beim erfindungsgemäßen Verfahren liegt darin, daß der vor der Bariumsulfat-Fällung entstandene Faktor IIa (Thrombin) am Bariumsulfat nicht gebunden wird, er also bereits hier vom Faktor X abgetrennt wird, während beim Faktor II das Gegenteil der Fall ist. Das erfindungsgemäße Verfahren hat also gegenüber dem Verfahren der DE—OS 26 54 189 den Vorteil, daß bereits mit einem Schritt (Bariumsulfat-Adsorption) ein weitgehend reines Faktor-X-Präparat erhalten wird. Eine u. U. vorzunehmende weitere Reinigung des aktivierten Faktor X über chromatographische Verfahren ist dann gegenüber dem Verfahren der DE—OS 26 54 189 natürlich wesentlich effektiver, da nur noch Spuren von Prothrombin (bzw. Thrombin) vorhanden sind. Die Aktivierung des Prothrombins vor der Bariumsulfat-Adsorption stellt somit einen wichtigen technischen Fortschritt bei der Isolierung reinen Faktor X (bzw. Xa) dar. Weiterhin hat sich gezeigt, daß in dieser Verfahrensstufe erfindungsgemäß der Faktor I mitentfernt wird. Das Vorliegen von Faktor I (Fibrinogen) im Prothrombin-Test wäre störend. Die auswahl der erfindungsgemäß verwendeten Komponenten und die angegebene Reaktionsfolge führte somit zu einem nicht zu erwartenden Ergebnis.

Weiterhin ist Gegenstand der Erfindung ein Reagenz zur Bestimmung von Prothrombin, enthaltend ein Thrombinsubstrat und einen Prothrombinaktivator, dadurch gekennzeichnet, daß der Prothrombinaktivator der Faktor Xa, vorzugsweise der menschliche Faktor Xa, ist.

Vorzugsweise besteht dieses Reagenz im wesentlichen aus Tris- und/oder Imidazol-Puffer, dem menschlichen Faktor Xa, als Prothrombinaktivator, wobei als Co-Reagentien vorteilhaft Phospholipide aus menschlichem Gehirn und Calciumchlorid verwendet werden, und einem synthetischen Thrombinsubstrat. Bevorzugt enthält ein Reagenz

— 0,3 bis 6,5 $\mu$g/ml Phospholipide,

— 0,7 bis 10 mM/l Calciumchlorid und

— 150 bis 380 $\mu$M/l Substrat,

wobei die Faktor-Xa-Konzentration 0,2—2,5% Plasmaextrakt entspricht. Das Reagenz kann in trockener und gelöster Form vorliegen.

Das erfindungsgemäße Verfahren und Reagenz ermöglichen eine rasche und zuverlässige Bestimmung des Prothrombins. Das Verfahren zeichnet sich aus durch seine Spezifität, weil der Faktor Xa nur normales Prothrombin und nicht auch PIVKA-Prothrombin erfaßt. Die bisher eingesetzten Aktivatoren wirken gerade nicht spezifisch. Durch die Zugabe eines Standardisierten Faktor-Xa-Präparats ist gewährleistet, daß in jedem Fall eine ausreichende Menge des Aktivators vorliegt. Es war sehr überraschend, daß der Faktor Xa nach Zusatz alles Prothrombin schnell und vollständig in Thrombin überführt, ohne, was sehr wesentlich ist, selbst in irgendeiner Weise an der Farbreaktion teilzunehmen. Es ist nämlich bekannt, daß der Faktor Xa z. B. auf synthetische Peptid-p-nitroanilid-Derivate, wie z. B. Chromozym TH, direkt spaltend einwirkt. Insbesondere war es auch überraschend,

daß der menschliche Faktor Xa vorteilhafter einsetzbar ist als z. B. Rinder-Faktor Xa. Es hat sich als ganz besonders vorteilhaft erwiesen, den Faktor Xa in definierter Menge zuzusetzen; die Aktivierung von an sich im Blutplasma vorhandenem Faktor Xa würde hier zu unbefriedigenden Ergebnissen führen. Weiterhin war es überraschend, daß durch ein so einfaches Herstellungsverfahren wie das erfindungsgemäße Verfahren ein Aktivator bereitgestellt werden kann, der die Ausführbarkeit, insbesondere die technische Brauchbarkeit gewährleistet, und bezüglich der Herkunft, nämlich dem menschlichen Plasma, überhaupt ausreichend ertragsfähig sein könnte.

Die folgenden Beispiele erläutern die Erfindung weiter.

Beispiel 1

*Herstellung eines Faktor-Xa-Präparats*

Zur Gewinnung des Normalplasmas wird Blut von gesunden Spendern mit einem Durchschnittsalter von 30 Jahren, je zur Hälfte von Frauen und Männern stammend, verwendet. Es enthält 25 mM Natriumcitrat. Das Plasma wird nach einer ersten Zentrifugation über 15 Min, 1500 g und Zimmertemperatur einer zweiten bei 4°C über 30 Minuten bei 20.000 g unterzogen. Pro 4 ml Normalplasma werden 1 ml/30 m wäßrige Calciumchlorid-Lösung und 2 Tropfen Echis carinatus venom (Sigma V 8250; Grundlösungen jeweils 1 mg/ml Wasser) hinzugefügt und etwa 2 Stunden im Wasserbad (37°C) belassen. Das entstandene Gerinnsel wird abzentrifugiert, und zum Überstand aus je 4 ml Normalplasma werden 150 mg Bariumsulfat zugesetzt. Es wird 30 Minuten lang bei Zimmertemperatur gerührt und zentrifugiert. Der Überstand wird verworfen und das Präzipitat wird viermal mit je ca. 4 ml physiologischer Kochsalzlösung gewaschen. Das Zentrifugat wird mit 2 ml einer 0,2 m Trinatriumcitrat-Lösung (pH 7,0) eluiert. Nach erneuter Zentrifugation wird der Überstand gegen physiologische Kochsalzlösung bei 4°C dialysiert. Das Dialysat wird in kleinen Protionen von etwa 250 µl bei −20°C aufbewahrt. Pro Portion werden bei Zimmertemperatur 30 µl einer 0,1 m Calciumchlorid-Lösung und 1 µl Russel's viper venom (Vipera Russelli, Wellcome) zugesetzt und etwa 14 Stunden bei 4°C stehengelassen. Dieses Präparat ist in den Test einsetzbar (2 µl entsprechend 1% Plasmaextrakt). Es kann lyophilisiert werden. Eine weitere Reinigung kann durchgeführt werden, wie auf Seite 5 ausführlich beschrieben ist.

Beispiel 2

*Methode der Prothrombin- und Thrombin-Bestimmung*

Es werden Testgemische hergestellt, welche enthalten:

| Substanz | Volumen | Endkonzentration |
|---|---|---|
| Puffer-Lösung | 400 µl minus andere eingesetzte Mengen | |
| Testplasma | 1—5 µl | 0.25—1.25% |
| Phospholipide | 2 µl | 1.25 µg/ml |
| Calciumchlorid-Lösung | 40 µl (0.1 m) | 10 mM/l |
| Aktivator (Faktor-Xa-Präparat gem. Beispiel 1) | 2 µl | entsprechend 1% Plasmafraktion |

Die Pufferlösung wird wie folgt hergestellt:

| | | |
|---|---|---|
| Stocklösung A: | 0,1 M/l Tris + 0,1 M/l | Imidazol + 0,1 M/l Salzsäure |
| Stocklösung B: | 0,1 M/l Tris + 0,1 M/l | Imidazol + 0,1 M/l Natriumchlorid |
| Stocklösung C: | 0,2 M/l Natriumchlorid | |

Der Gebrauchspuffer (pH = 8,4) wird hergestellt durch Vereinigung der Stocklösungen im Volumen-Verhältnis A:B:C = 1:1:2. Bei den verwendeten Phospholipiden handelt es sich um eine Aceton/Äther-Extraktion aus menschlichem Gehirn nach W. N. Bell and H. G. Alton, Nature (London) 174, Seite 880 (1954).

Die Komponenten des Testgemisches werden direkt in der angegebenen Reihenfolge in eine Mikroküvette mit Schichtdicke 1 cm eines Spectrophotometers "Aminco DW 2" bei 37°C pipettiert. Die Inkubationszeit für den Faktor Xa beträgt 120 Sekunden. Es werden dann 50 µl (1,5 mM/l) Substrat (Chromozym TH, Boehringer Mannheim GmbH, oder Substrat 2238, Kabi) zugegeben, wobei die Endkonzentration 187,5 µM/l (Chromozym TH) bzw. 148 µM/l (S 2238) beträgt. Nach möglichst raschem, gutem Mischen erfolgt sofortiger Meßbeginn.

Die Absorption pro Zeit wird direkt aufgezeichnet mit einer Zeigerlaufgeschwindigkeit in Minuten pro cm in der Horizontalen und einer Absorption in mm pro mm full scale in der Vertikalen. Hierbei können verschiedene Absorptionsempfindlichkeiten und Laufgeschwindigkeiten gewählt werden. Die Absorptionsänderung pro Minute errechnet sich daher nach folgender Formel:

$$\frac{\dfrac{\text{gemessene Absorption in mm}}{\text{Gesamtscala in mm}} \times \text{Absorptionsempfindlichkeit}}{\text{Zeigerlaufgeschwindigkeit in min/cm} \times \text{Meßstrecke}}$$

*Beispiel* für Meßstrecke 10 cm, Vertikale 43 mm full scale 252 mm, Absorptionsempfindlichkeit 0,5 und Laufgeschwindigkeit 0,033:

$$\frac{\dfrac{43}{252} \times 0{,}5}{0{,}033 \times 10\ \text{min/cm}} = 0{,}258 \quad \frac{\text{Absorptionsänderung}}{\text{Minute}}$$

## Patentansprüche

1. Verfahren zur Bestimmung von Prothrombin in biologischen Material, wie Blutplasma, durch Umwandlung in Thrombin, enzymatische Spaltung eines Thrombinsubstrats mit dem Thrombin und Messung eines Spaltprodukts, dadurch gekennzeichnet, daß zu dem zu untersuchenden biologischen Material Faktor Xa, vorzugsweise menschlicher Faktor Xa, zugegeben wird.

2. Verfahren nach Anspruch 1, dadurch gekennzeichnet, daß das Thrombinsubstrat ein synthetisches Peptid-p-nitroanilid-Derivat, insbesondere N-Tos-Gly-Pro- Arg-pNA, N-Cbz-Gly-Pro-Arg-pNA, H-D-Phe-Pip-Arg-pNA oder Bz-phe-Val-Arg-pNA ist.

3. Reagenz zur Bestimmung von Prothrombin in biologischem Material, wie Blutplasma, enthaltend ein synthetisches Thrombinsubstrat und einen Prothrombinaktivator, dadurch gekennzeichnet, daß der Prothrombinaktivator der Faktor Xa, vorzugsweise der menschliche Faktor Xa, ist.

4. Reagenz zur Bestimmung von Prothrombin, im wesentlichen bestehend aus Tris- und/oder Imidazol-Puffer, dem Faktor Xa, vorzugsweise dem menschlichen Faktor Xa, Co-Reagentien, wie Phospholipide und Calciumchlorid, und einem synthetischen Thrombinsubstrat, wie N-Tos-Gly-Pro-Arg-pNA, N-Cbz-Gly-Pro-Arg-pNA, H-D-Phe-Pip-Arg-pNA oder Bz-Phe-Val-Arg-pNA.

5. Reagenz nach Anspruch 4, dadurch gekennzeichnet, daß es 0,3 bis 6,5 $\mu$g/ml Phospholipide, 0,7 bis 10 mM/l Calciumchlorid und 150 bis 380 $\mu$M/l Thrombinsubstrat enthält und die Faktor-Xa-Konzentration 0,2—2,5% Plasmaextrakt entspricht.

6. Verfahren zur Herstellung eines Faktor-Xa-Präparats, dadurch gekennzeichnet, daß man Plasma, vorzugweise menschliches Plasma, mit einem Prothrombinaktivator, vorzugsweise Echis-carinatus-venom, behandelt, nach Zentrifugieren mit einem Proteinadsorbens, vorzugsweise Bariumsulfat, versetzt, das Präzipat mit einem Proteinelutionsmittel, vorzugsweise einer wäßrigen Natriumcitrat-Lösung, eluiert und das Eluat mit einem Faktor-X-Aktivator, vorzugsweise Russel's viper venom, und gegebenenfalls einem löslichen Calciumsalz, vorzugsweise Calciumchlorid, versetzt.

7. Verfahren nach Anspruch 6, dadurch gekennzeichnet, daß, für den Fall, daß die Elution mit Proteinelutionsmitteln durchgeführt wird, die Calcium-bindende Komponenten, wie Citrat und Oxalat, enthalten, das Eluat dialysiert wird.

8. Verfahren nach einem der Ansprüche 6 und 7, dadurch gekennzeichnet, daß eine weitere Reinigung des erhaltenen Faktor-Xa-Präparats erfolgt durch Gelfiltration mit einem Molekularsieb und/oder Ammoniumsulfat-Präzipitation und/oder Ionenaustauscher-Chromatographie und/oder präparative Elektrophorese und/oder Ultrazentrifugation.

## Claims

1. Process for the determination of prothrombin in biological material, such as blood plasma, by conversion into thrombin, enzymatic fission of a thrombin substrate with the thrombin and measurement of a fission product, characterised in that Factor Xa, preferably human Factor Xa, is added to the biological material to be investigated.

2. Process according to claim 1, characterised in that the thrombin substrate is a synthetic piptide *p*-nitroanilide derivative, especially N-Tos-Gly-Pro-Arg-pNA, N-Cbz-Gly-Pro-Arg-pNA, H-D-Phe-Pip-Arg-pNA or Bz-Phe-Val-Arg-pNA.

3. Reagent for the determination of prothrombin in biological material, such as blood plasma, containing a synthetic thrombin substrate and a prothrombin activator, characterised in that the prothrombin activator is the Factor Xa, preferably the human Factor Xa.

4. Reagent for the determination of prothrombin, consisting essentially of tris and/or imidazole buffer, the Factor Xa, preferably the human Factor Xa, coreagents, such as phospholipids and calcium chloride, and a synthetic thrombin substrate, such as N-Tos-Gly-Pro-Arg-pNA, N-Cbz-Gly-Pro-Arg-pNA, H-D-Phe-Pip-Arg-pNA or Bz-Phe-Val-Arg-pNA.

5. Reagent according to claim 4, characterised in that it contains 0.3 to 6.5 $\mu$g/ml of phospholipids, 0.7 to 10 mM/liter calcium chloride and 150 to 380 $\mu$M/litre thrombin substrate and the Factor Xa concentration corresponds to 0.2—2.5% of plasma extract.

6. Process for the production of a Factor Xa preparation, characterised in that one treats plasma, preferably human plasma, with a prothrombin activator, preferably *Echis carinatus* venom, after centrifuging mixes with a protein adsorbent, preferably barium sulphate, elutes the precipitate with a protein elution agent, preferably an aqueous sodium citrate solution, and mixes the eluate with a Factor X activator, preferably Russell's viper venom, and possibly with a soluble calcium salt, preferably calcium chloride.

7. Process according to claim 6, characterised in that, for the case in which the elution is carried out with protein elution agents which contain calcium-binding components, such as citrate and oxalate, the eluate is dialysed.

8. Process according to one of claims 6 and 7, characterised in that a further purification of the Factor Xa preparation obtained takes place by gel filtration with a molecular sieve and/or ammonium sulphate precipitation and/or ion exchange chromatography and/or preparative electrophoresis and/or ultra-centrifuging.

**Revendications**

1. Procédé pour le dosage de prothrombine dans des milieux biologiques tels que le plasma sanguin, par transformation en thrombine, lyse enzymatique d'un substrat de thrombine avec la thrombine et mesure d'un des produits ainsi formés, caractérisé en ce que l'on ajoute au milieu biologique à examiner du facteur Xa, de préférence le facteur Xa humain.

2. Procédé selon la revendication 1, caractérisé en ce que le substrat de thrombine est un dérivé d'un p-nitro-anilide d'un peptide synthétique, en particulier le N-Tos-Gly-Pro-Arg-pNA, le N-Cbz-Gly-Pro-Arg-pNA, le H-O-Phe-Pip-Arg-pNA ou le Bz-phe-Val-Arg-pNA.

3. Agent pour le dosage de prothrombine dans des milieux biologiques, tel le plasma sanguin, comprenant un substrat de thrombine synthétique et un activateur de la prothrombine, caractérisé en ce que cet activateur de la prothrombine est le facteur Xa, de préférence le facteur Xa humain.

4. Agent pour le dosage de prothrombine, comprenant essentiellement un tampon de tris et/ou d'imidazole, le facteur Xa, de préférence le facteur Xa humain, des réactifs-Co, comme les phospholipides et le chlorure de calcium, et un substrat de thrombine synthétique, tel que le N-Tos-Gly-Pro-Arg-pNA, le N-Cbz-Gly-Pro-Arg-pNA, le H-D-Phe-Pip-Arg-pNA ou le Bz-Phe-Val-Arg-pNA.

5. Agent selon la revendication 4, caractérisé en ce qu'il comprend entre 0,3 et 6,5 mg/ml de phospholipides, entre 0,7 et 10 mmole/l de chlorure de calcium et de 150 à 380 $\mu$mole/l de substrat de thrombine et en ce que la concentration du facteur Xa correspond à une proportion comprise entre 0,2 et 2,5% d'extrait de plasma.

6. Procédé pour l'obtention d'une préparation du facteur Xa caractérisé en ce que l'on traite du plasma, de préférence de provenance humaine, avec un activateur de prothrombine, de préférence de l'echis-carinatus-venom, et auquel on ajoute après centrifugation un absorbant de protéines, de préférence du sulfate de baryum, on élue le précipité avec un éluant pour protéines, de préférence une solution aqueuse de citrate de sodium, et on ajoute à l'éluat un activateur de facteur X, de préférence le vénin de vipères selon Russel et éventuellement un sel de calcium soluble, de préférence le chlorure de calcium.

7. Procédé selon la revendication 6, caractérisé en ce que dans le cas où l'on effectue l'élution avec des éluants pour protéines contenant des composants liant le calcium, tels que le citrate et l'oxalate, on soumet l'éluat à une dialyse.

8. Procédé selon l'une quelconque des revendications 6 et 7, caractérisé en ce qu'une purification complémentaire de la préparation du facteur Xa est réalisée au moyen d'une filtration sur gel avec un tamis moléculaire et/ou un précipité de sulfate d'ammonium et/ou par chromatographie sur échangeur d'ions et/ou par électrophorèse préparative et/ou par ultracentrifugation.